Europäisches Patentamt

(19) European Patent Office

Office européen des brevets

(11) Numéro de publication: **0 055 657**
**A1**

## (12) DEMANDE DE BREVET EUROPEEN

(21) Numéro de dépôt: **81402022.8**

(22) Date de dépôt: **17.12.81**

(51) Int. Cl.³: **A 61 B 5/14**

(30) Priorité: **20.12.80 GB 8040916**

(43) Date de publication de la demande:
**07.07.82 Bulletin 82/27**

(84) Etats contractants désignés:
**AT BE CH DE FR GB IT LI LU NL SE**

(71) Demandeur: **Feaster, William Wardock**
**31 avenue Princesse Grace**
**Monte Carlo(MC)**

(71) Demandeur: **Feaster, Danielle Gugliere**
**31 avenue Princesse Grace**
**Monte Carlo(MC)**

(72) Inventeur: **Feaster, William Wardock**
**31 avenue Princesse Grace**
**Monte Carlo(MC)**

(72) Inventeur: **Feaster, Danielle Gugliere**
**31 avenue Princesse Grace**
**Monte Carlo(MC)**

(74) Mandataire: **Bonnetat, Christian et al,**
**Cabinet PROPI Conseils 23 rue de Léningrad**
**F-75008 Paris(FR)**

(54) **Système de prélèvement de liquide physiologique, tel que du sang.**

(57) Système de prélèvement de liquide physiologique, tel que du sang comportant des moyens d'aspiration manuelle ou mécanique pourvus d'un cylindre 6 et d'une aiguille 7.

Ce système comporte au moins un récipient tubulaire (3) susceptible d'être maintenu dans le prolongement du cylindre (6), du côté de l'aiguille (7), par des moyens de retenue (4) portés par ledit cylindre (6), de manière que le fond (8) dudit récipient (3) soit traversé de façon étanche par ladite aiguille (7) ledit récipient (3) étant pourvu d'un capuchon (9,9',9'') obturant son extrémité opposée à ladite aiguille (7), et portant un organe de prélèvement dudit liquide (9'a,10).

Fig.1

EP 0 055 657 A1

1

## Système de prélèvement de liquide physiologique, tel que du sang.

La présente invention concerne un système de prélèvement de liquide physiologique, tel que du sang, et plus particulièrement un système permettant le prélèvement d'échantillons de faible volume.

De façon connue, le prélèvement de sang, notamment en vue de la constitution d'échantillons, est effectué à l'aide d'une seringue à aiguille, la traction du piston entraînant l'aspiration du sang dans le corps de la seringue.

Dans le cas où la quantité de sang prélevé est destinée à subir des opérations d'analyse ou de test, on doit, de façon connue, transférer le volume de sang contenu dans le corps de la seringue dans un ou plusieurs récipients selon le nombre d'opérations à effectuer.

Ce transfert entraîne des risques de contamination (par exemple en cas d'hépatite) de l'opérateur par le sang éventuellement infecté avec les conséquences graves que cette situation implique. Ce risque de contamination est d'autant plus élevé que le nombre d'analyses, donc de récipients différents, est plus grand.

En outre, ce transfert du sang prélevé, du corps de la seringue dans le ou les récipients, est susceptible de donner lieu à des erreurs ou à des difficultés de manipulations, notamment dans le cas de faibles volumes.

De plus, grâce aux perfectionnements des techniques et du matériel d'analyse sanguine, on peut maintenant obtenir des analyses et tests satisfaisants à partir de très faibles quantités de sang. Dans de nombreux cas, on peut prélever suffisamment de sang par la méthode capillaire (bout de doigt, oreille, etc...), qui met en oeuvre des tubes capillaires. Le contenu de ces tubes, après prélèvement, doit être

2

transféré dans un autre récipient à des fins d'analyse. On conçoit qu'il est très difficile de prélever et transférer de façon précise de petits volumes de sang avec une seringue classique, et que cela est même impossible avec de très petits volumes.

La présente invention remédie à ces inconvénients, et concerne un système de prélèvement de sang (aussi bien capillaire que veineux) de mise en oeuvre simple, permettant d'obtenir un volume de sang faible et correspondant exactement à la quantité désirée, les risques de contamination étant limités, du fait que le sang prélevé est introduit dans le récipient qui est directement utilisé pour réaliser les analyses et les tests envisagés.

A cette fin, selon l'invention, le système de prélèvement de liquide physiologique, tel que du sang, comportant des moyens d'aspiration manuelle ou mécanique pourvus d'un cylindre et d'une aiguille, est caractérisé en ce qu'il comporte au moins un récipient tubulaire susceptible d'être maintenu dans le prolongement du cylindre, du côté de l'aiguille, par des moyens de retenue portés par ledit cylindre, de manière que le fond dudit récipient soit traversé de façon étanche par ladite aiguille, ledit récipient étant pourvu d'un capuchon obturant son extrémité opposée à ladite aiguille et pourvu d'un organe de prélèvement dudit liquide.

On voit que, dans le système selon l'invention, ladite aiguille sert de connexion entre les moyens d'aspiration et le récipient lorsque celui-ci est maintenu par les moyens de retenue et que l'aiguille traverse le fond dudit récipient, ce qui permet aux moyens d'aspiration d'aspirer l'air contenu dans le récipient et le liquide amené par l'organe de prélèvement.

Ainsi, grâce au système selon l'invention, on évite le risque de contamination puisque le sang prélevé passe directement du patient dans le récipient qui sera utilisé pour effectuer les analyses ou tests nécessaires. En outre, la désolidarisation du récipient et des moyens d'aspiration n'affecte ni l'étanchéité du récipient, ni l'utilisation ultérieure desdits moyens d'aspiration. On remarquera que pendant le prélèvement le cylindre des moyens d'aspiration sert de moyen de préhension et de manipulation dudit récipient, qui peut alors être petit.

Suivant que l'on désire prélever du sang veineux ou capillaire, l'organe de prélèvement dont est doté ledit capuchon est soit une aiguille, soit un tube capillaire.

Afin de réaliser un dispositif d'utilisation pratique, les moyens de retenue sont constitués d'un organe tubulaire dans lequel s'ajuste ledit récipient de façon amovible.

On notera que cet organe tubulaire entoure l'aiguille des moyens d'aspiration et peut servir à protéger, d'une part l'opérateur contre les piqûres accidentelles par l'aiguille, d'autre part, l'aiguille contre d'éventuelles détériorations.

Ledit organe tubulaire constituant les moyens de retenue du récipient est de préférence solidarisé du cylindre d. manière amovible (par exemple par vissage ou par friction), notamment pour faciliter l'éventuel changement de l'aiguille des moyens d'aspiration.

Cependant, cet organe tubulaire pourrait également être solidarisé du cylindre de manière définitive, ledit organe pouvant alors être constitué par le prolongement de la paroi du cylindre.

Lors de l'aspiration du sang par les moyens d'aspiration dans le cylindre, il est bien entendu nécessaire que le fond du récipient soit traversé par l'aiguille desdits moyens d'aspiration de manière étanche. Le fond doit en outre être étanche, lorsque ce dernier est désolidarisé des

4

moyens d'aspiration pour effectuer les analyses ; autrement dit le sang contenu ne doit pas pouvoir sortir dudit récipient.

A cet effet, le fond du récipient comporte une cloison, en retrait de l'extrémité inférieure dudit récipient, qui est pourvue d'une lumière de diamètre suffisamment grand pour permettre le passage de l'aiguille des moyens d'aspiration et contre laquelle s'applique une membrane souple en un matériau élastique, tel que du caoutchouc, disposée du côté extérieur de la cloison et destinée à être percée par ladite aiguille, ladite membrane présentant une élasticité suffisante pour permettre, après enlèvement de l'aiguille, l'obturation de l'ouverture créée par l'enfoncement de ladite aiguille.

On obtient ainsi l'étanchéité aussi bien lorsque l'aiguille des moyens d'aspiration traverse la membrane, que lorsque ladite aiguille est retirée, lors de la désolidarisation du récipient et de ces moyens d'aspiration. De plus, dans le cas où le sang prélevé doit subir une centrifugation risquant d'entraîner une déformation de la membrane susceptible de provoquer ainsi un écartement des lèvres de l'ouverture réalisé lors de l'introduction de l'aiguille de la seringue dans ladite membrane, il est avantageux que le fond comporte en outre un bouchon percé permettant le passage de l'aiguille des moyens d'aspiration et comportant une surface concave en regard de la membrane élastique et servant d'appui à celle-ci lorsque le récipient est soumis à de fortes accélérations, de façon à fermer l'ouverture percée par l'aiguille.

La cloison, la membrane et le bouchon sont solidarisés du récipient, par exemple par collage, soudage par ultra-sons etc... On notera que ledit bouchon a également pour fonction d'empêcher la membrane de s'échapper pendant la centrifugation.

Les figures du dessin annexé feront bien comprendre comment l'invention peut être réalisée.

La figure 1 est une vue en coupe d'un mode de réalisation du système selon l'invention.

La figure 2 est une vue en coupe du système montré à la figure 1, le récipient étant ôté.

La figure 3 illustre en coupe le récipient de base selon l'invention, non muni de son dispositif d'étanchéité.

Les figures 4 et 5 sont des coupes d'exemples de réalisation du récipient, utilisant des récipients semblables ou identiques au récipient de base de la figure 3 associé à des dispositifs d'étanchéité.

Les figures 6 à 8 sont des coupes d'exemples de réalisation de capuchon pour ledit récipient.

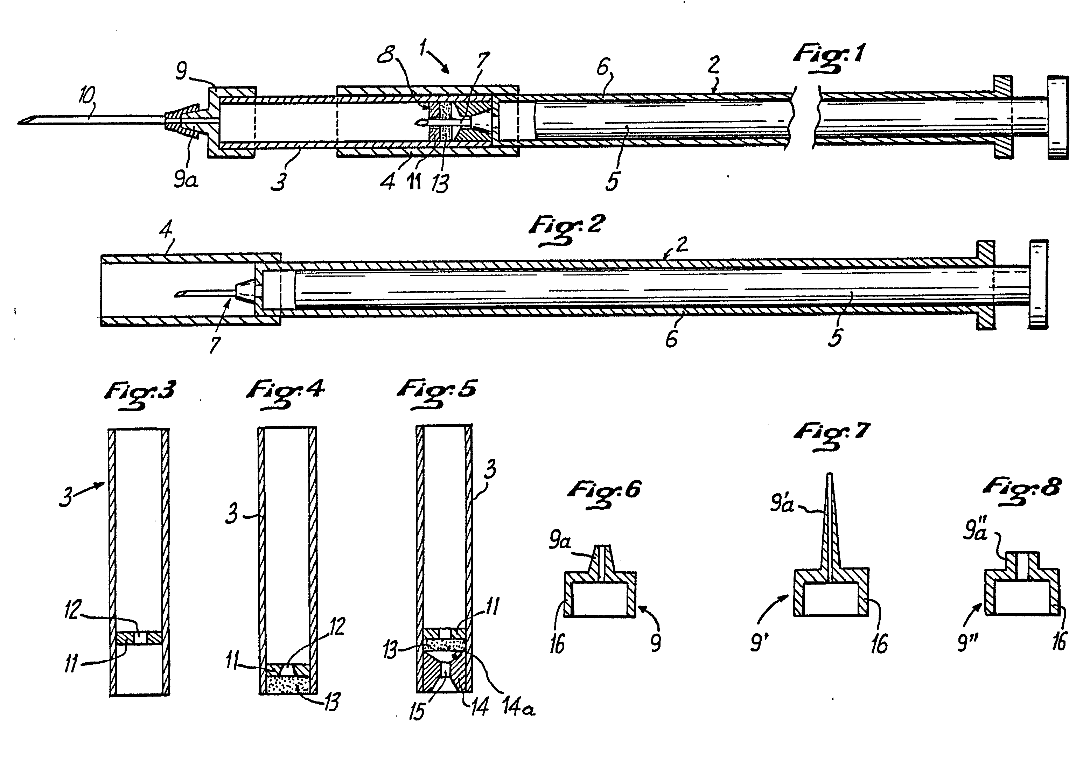

L'exemple de réalisation 1 du système selon l'invention pour le prélèvement de sang, montré sur la figure 1, comporte une seringue 2 et un récipient tubulaire 3 solidarisé de cette dernière par des moyens de retenue tel qu'un tube 4. La seringue 2 est composée de manière connue d'un piston 5, coulissant dans le cylindre 6 et d'une aiguille 7. L'aiguille 7 est de préférence amovible pour pouvoir être remplacée, dans le cas où elle deviendrait émoussée après de nombreux usages.

Le récipient tubulaire 3 est disposé dans le prolongement de la seringue 2 du côté de l'aiguille 7.

Dans l'exemple de réalisation montré sur les figures, le récipient 3 et le cylindre 6 ont un diamètre sensiblement égal.

Le tube de retenue 4 est solidarisé de manière amovible du cylindre 6, afin de faciliter le remplacement éventuel de l'aiguille 7.

Le récipient 3 comporte un fond 8 apte à être traversé de manière étanche par l'aiguille 7, (qui participe ainsi à la fixation du récipient 3 sur la seringue 2), et un capuchon amovible 9 pourvu d'un embout 9a sur lequel est montée une aiguille 10 de prélèvement, pénétrant par exemple dans une veine du patient.

Le volume du récipient 3 correspond au volume de sang nécessaire pour effectuer l'analyse envisagée.

La conception des différents éléments du système selon l'invention, solidarisés de manière amovible autorise une grande souplesse d'utilisation et d'interchangeabilité.

Dans un premier temps, l'opérateur introduit le récipient 3 dans le tube 4 jusqu'à ce que l'aiguille 7 traverse le fond 8. Le récipient 3 est pourvu de son capuchon 9 et de l'aiguille de prélèvement 10.

Le piston 5 est poussé à fond, puis après introduction de l'aiguille 10 dans une veine du patient, le piston est soumis à une traction continue aspirant l'air du récipient 3, puis le sang, et le récipient 3 est rempli jusqu'à un niveau pour lequel de l'air reste interposé entre le sang et l'aiguille 7. Le récipient 3 est alors désolidarisé de la seringue 2 afin de permettre la réalisation des tests et analyses nécessaires. L'étanchéité du fond 8 est obtenue grâce à la conception particulière de ce dernier comme il est décrit ci-après.

Le fond 8 du récipient 3 comporte (comme le montre la figure 3 sur laquelle le dispositif d'étanchéité n'a pas été représenté), une cloison 11 pourvue d'une lumière 12 de diamètre suffisamment grand pour permettre le passage de l'aiguille 7 de la seringue 2 et disposée en retrait de l'extrémité inférieure dudit récipient.

Dans l'exemple de réalisation du récipient 3 montré sur la figure 4, le fond comporte en outre une membrane souple élastique 13, en caoutchouc par exemple, disposée contre la cloison 11 du côté opposé au volume intérieur du récipient 3. La membrane 13 en caoutchouc assure une étanchéité très satisfaisante, aussi bien lorsque l'aiguille 7 traverse ladite membrane 13 que lorsque l'aiguille est retirée, lors de ladite désolidarisation du récipient 3 de la seringue 2.

Il peut s'avérer nécessaire dans certains cas, par exemple lorsque l'échantillon sanguin doit être soumis à une centrifugation que le fond 8 du récipient 3 soit particulièrement étanche. Dans ce cas, le récipient 3 (désolidarisé de la seringue 2) est soumis à de fortes accélérations et la membrane 13 risquerait peut-être d'être déformée par les forces centrifuges, ce qui provoquerait l'écartement des lèvres du trou percé par l'aiguille 7, dans la membrane 13 et donc entraînerait des fuites du contenu liquide du récipient 3.

C'est pourquoi le fond 8 du récipient 3 comporte alors avantageusement (voir la figure 5) un bouchon 14, percé d'une ouverture 15 apte à laisser libre passage à l'aiguille 7 de la seringue 2 et permettant de limiter la déformation de la membrane 13 soumise à des accélérations centrifuges. A cet effet, le bouchon 14 comporte une surface concave 14a en regard de la membrane 13. Ainsi, pendant la centrifugation la membrane est appliquée par les forces centrifuges contre la surface concave 14a de sorte qu'il en résulte une constriction supplémentaire du trou percé par l'aiguille 7 dans la membrane 13.

Le capuchon 9, montré sur la figure 1 et destiné à être pourvu de l'aiguille de prélèvement 10 (voir également la figure 6) comporte une partie réceptrice 16 de l'extrémité du récipient 3, et un embout 9a pour la mise en place de l'aiguille de prélèvement 10.

8

Dans certains cas (par exemple prélèvement de sang sur un enfant), il est préférable de ne pas utiliser d'aiguille 10 et d'aspirer du sang provenant d'un vaisseau capillaire. On utilise alors un capuchon 9' (voir la figure 7) à la place du capuchon 9. Ce capuchon 9' comporte également une partie réceptrice 16 de l'extrémité du récipient 3, mais l'embout 9a d'adaptation de l'aiguille 10 est remplacé par un embout d'aspiration capillaire 9'a. Sur la figure 8, on a représenté un capuchon 9" dont la partie 16 comporte un embout 9"a auquel peut être adapté un tube capillaire d'aspiration séparé (non représenté).

L'invention n'est bien entendu pas limitée aux seuls exemples de réalisation décrits en regard des figures. Notamment, les moyens de retenue du récipient 3 sur le cylindre 6 sont susceptibles d'être réalisés sous la forme de doigts solidaires de l'extrémité dudit cylindre, débordant de celui-ci et aptes à enserrer le récipient 3, ou bien encore par le prolongement dudit cylindre. De même, au lieu d'être manuels, les moyens d'aspiration 2 pourraient être mécaniques.

Il est avantageux pour des raisons de sécurité que les moyens de retenue débordent de la seringue sur une longueur supérieure à celle de l'aiguille 7 faisant saillie. Des moyens d'obturation amovibles (non représentés) de l'extrémité ouverte du tube de retenue 4 (de la figure 1) peuvent être prévus pour permettre de conserver la seringue et notamment son aiguille 7, dans une atmosphère protégée.

REVENDICATIONS

1.- Système de prélèvement de liquide physiologique, tel que du sang comportant des moyens d'aspiration manuelle ou mécanique pourvus d'un cylindre 6 et d'une aiguille 7, caractérisé en ce qu'il comporte au moins un récipient' tubulaire (3) susceptible d'être maintenu dans le prolongement du cylindre (6) , du côté de l'aiguille (7), par des moyens de retenue (4) portés par ledit cylindre (6), de manière que le fond (8) dudit récipient (3) soit traversé de façon étanche par ladite aiguille (7) ledit récipient (3) étant pourvu d'un capuchon (9,9',9") obturant son extrémité opposée à ladite aiguille (7) et portant un organe de prélèvement dudit liquide (9'a,10).

2.- Système selon la revendication 1, caractérisé en ce que les moyens de retenue sont constitués d'un organe tubulaire (4) dans lequel s'ajuste ledit récipient (3) de façon amovible.

3.- Système selon la revendication 2, caractérisé en ce que l'organe tubulaire (4) est solidarisé de manière amovible du cylindre (6).

4.- Système selon l'une des revendications 1 à 3, caractérisé en ce que le fond (8) du récipient (3) comporte une cloison (11), en retrait de l'extrémité inférieure du récipient (3) qui est pourvue d'une lumière (12) de diamètre suffisamment grand pour permettre le passage de l'aiguille (7) des moyens d'aspiration et contre laquelle s'applique une membrane (13) souple en un matériau élastique, tel que du caoutchouc, disposée du côté extérieur de la cloison (11) et destinée à être percée par ladite aiguille, ladite membrane (13) présentant une élasticité suffisante pour permettre, après enlèvement de l'aiguille (7) l'obturation de l'ouverture créée par l'enfoncement de ladite aiguille (7).

5.-Système selon la revendication 4, caractérisé en ce que le fond (8) comporte un bouchon percé (14) permettant le passage de l'aiguille (7) et présentant une surface concave (14a) servant d'appui à la membrane élastique (13) lorsque le récipient est soumis à de fortes accélérations, de façon à fermer l'ouverture percée par l'aiguille (7).

6.- Système selon l'une quelconque des revendications 1 à 5, caractérisé en ce que ledit organe de prélèvement (9'a) est solidaire du capuchon (9').

7.- Système selon l'une quelconque des revendications 1 à 5, caractérisé en ce que ledit organe de prélèvement (10) est monté de façon amovible sur le capuchon (9).

0055657

Fig.1  Fig.2  Fig.3  Fig.4  Fig.5  Fig.6  Fig.7  Fig.8

**Office européen des brevets**

## RAPPORT DE RECHERCHE EUROPEENNE

EP 81 40 2022

| | DOCUMENTS CONSIDERES COMME PERTINENTS | | CLASSEMENT DE LA DEMANDE (Int. Cl. ³) |
|---|---|---|---|
| Catégorie | Citation du document avec indication, en cas de besoin, des parties pertinentes | Revendica-tion concernée | A 61 B 5/14 |
| A | FR - A - 459 661 (F. MALOINE)<br><br>* page 1, ligne 1 à page 2, ligne 19; figures 1-3 *<br>--- | 1,7 | |
| A | FR - A - 2 409 040 (J.L. REBILLARD)<br><br>* page 1, ligne 14 à page 2, ligne 16; figures 1 et 2 *<br>--- | 1,2,4 | |
| A | US - A - 4 140 108 (E.L. NUGENT/ BECTON, DICKINSON and COMP.)<br><br>* résumé; colonne 2, ligne 34 à colonne 3, ligne 19; figure 1 *<br>--- | 1 | **DOMAINES TECHNIQUES RECHERCHES (Int. Cl. ³)**<br><br>A 61 B 5/14<br>G 01 N 1/00<br>A 61 M 5/00 |
| A | FR - A - 1 305 752 (ENGIS EQUIPMENT COMP.)<br><br>* page 2, colonne de gauche, lignes 15-19; figure 1 *<br>--- | 6 | |
| A | FR - A - 2 399 848 (BECTON, DICKINSON AND COMP.)<br><br>* page 6, ligne 12 à ligne 38; figures 1-5 *<br>--- | 4,5 | . |
| A | US - A - 3 965 889 (C.E. SACHS/ COMMISSARIAT A L'ENERGIE ATOMIQUE)<br><br>* colonne 3, ligne 32 à ligne 41; figures 1 et 2 *<br>--- | 4,5 | **CATEGORIE DES DOCUMENTS CITES**<br><br>X: particulièrement pertinent à lui seul<br>Y: particulièrement pertinent en combinaison avec un autre document de la même catégorie<br>A: arrière-plan technologique·<br>O: divulgation non-écrite<br>P: document intercalaire<br>T: théorie ou principe à la base de l'invention<br>E: document de brevet antérieur, mais publié à la date de dépôt ou après cette date<br>D: cité dans la demande<br>L: cité pour d'autres raisons |
| A | US - A - 3 785 367 (R.F. FORTIN et al./ PHARMASEL DIVISION OF AMERICAN HOSPITAL SUPPLY CORP.)<br><br>* colonne 2, lignes 10-14; figure 1 *<br>------------ | 7 | |
| | Le présent rapport de recherche a été établi pour toutes les revendications | | &: membre de la même famille, document correspondant |

| Lieu de la recherche | Date d'achèvement de la recherche | Examinateur |
|---|---|---|
| La Haye | 24.02.1982 | ZILLIOX |